# EUROPEAN PATENT APPLICATION

(11) **EP 2 540 834 A1**
(43) Date of publication of application: **02.01.2013**
(21) Application number: 11305828.3
(22) Date of filing: 29.06.2011
(51) Int. Cl.: C12P 7/18, C12N 9/00, C12P 7/04, C12P 1/02

(54) **Method for the preparation of 1,3-propanediol**

(71) Applicant: Metabolic Explorer, 63360 Saint Beauzire (FR)
(72) Inventor: Boisart, Cédric, 63360 Gerzat (FR)
(74) Representative: Regimbeau

(57) **Abstract**

The present invention concerns a microorganism genetically modified for the bioproduction of 1,3-propanediol, wherein the microorganism comprises :

- a three-step metabolic pathway for the production of 1,3-propanediol, comprising a first step of conversion of L-homoserine to 4-hydroxy-2-ketobutyrate, a second step of decarboxylation of 4-hydroxy-2-ketobutyrate to 3-hydroxypropionaldehyde with an enzyme having a hydroxy-2-keto acid decarboxylase activity, and a third step of reduction of 3-hydroxypropionaldehyde to 1,3-propanediol with an enzyme having hydroxy aldehyde reductase activity, and
- overexpression of a gene encoding a pyruvate carboxylase.

The invention also concerns a new method for the biological preparation of 1,3-propanediol by fermentation, comprising cultivating said microorganism genetically modified, wherein the culture is performed in an appropriate medium comprising a source of carbon, and recovering the 1,3-propanediol being produced.

## Description

The present invention relates to a new microorganism and method for the biological preparation of 1,3-propanediol. In particular, the present invention provides a microorganism comprising a three-step metabolic pathway for the production of 1,3-propanediol and overexpression of a gene encoding a pyruvate carboxylase.

### BACKGROUND

Fermentative production of 1,3-propanediol by culturing microorganism producing 1,3-propanediol is known in the art. However, these methods generally involve vitamin B12-dependent enzymes such as disclosed in patent application WO1998021339 and WO2004033646, which make the production of 1,3-propanediol very expensive.

Accordingly, there is a need for alternative solutions to the production of 1,3-propanediol through vitamin B12-independent pathways from renewable sources of carbon.

1,3-propanediol may be produced from glycerol by using a vitamin B12-independent enzyme as described, for example, in the patent application W02010128070.

The inventors have designed a new 1,3-propanediol biosynthetic pathway from other renewable sources than glycerol that does not involve vitamin B12. This new 1,3-propanediol biosynthetic pathway allows the production of 1,3-propanediol from glucose or saccharose via the intermediate oxaloacetate through at least six enzymatic reactions as described in patent applications WO2010076324, US61/361,459 and EP10305729. Among these six enzymatic reactions, three involved enzymes are not specific to the substrates engaged.

### PRIOR ART

Many microorganisms have developed 'anaplerotic pathways' that regenerate intermediates of the tricarboxylic acid cycle (TCA) cycle. In particular, oxaloacetate can be formed from phosphoenol-pyruvate or from pyruvate. These pathways can be utilized by microorganisms under either aerobic or anaerobic conditions. The conversion of pyruvate into oxaloacetate is catalyzed by the enzyme called 'pyruvate carboxylase'.

The patent application WO19995303 describes a method for increasing the carbon flow toward oxaloacetate through the overexpression of pyruvate carboxylase, in order to enhance production of biochemicals derived from oxaloacetate such as lysine, threonine and succinate.

The patent application WO1999018228 describes a method for increasing the production of amino acids belonging to the aspartate and/or glutamate family through the overexpression of pyruvate carboxylase, in particular threonine, homoserine and glutamate.

The prior art provides no information on the possibility of improving the production of any other molecule than TCA cycle constituents, amino acids or nucleotides.

The metabolic pathway including oxaloacetate is distantly upstream of the reactions producing 1,3-propanediol, and is mainly connected to the TCA cycle and to the production of amino acids as described above. Accordingly, there was no evidence that an enhanced production of oxaloacetate may have an impact on the production of 1,3-propanediol obtained through the pathway of the invention, in addition to the impact on the production of amino acids, nucleotides and on providing acceptor molecules for the TCA cycle.

Surprisingly, the inventors found that enhancing pyruvate carboxylase activity leads to an improved production of 1,3-propanediol.

### GENERAL DESCRIPTION OF THE INVENTION

The inventors of this application have previously described the production of 1,3-propanediol through a specific pathway comprising three enzymatic reactions starting with the transformation of an L-homoserine precursor. First, a homoserine transaminase or an enzyme having homoserine oxidase activity or any equivalent enzyme having capability to convert L-homoserine into 4-hydroxy-2-ketobutyrate promotes the synthesis of 4-hydroxy-2-ketobutyrate. Second, an enzyme having hydroxy-2-keto acid decarboxylase activity promotes the conversion of 4-hydroxy-2-ketobutyrate into 3-hydroxypropionaldehyde. Finally, 3-hydroxypropionaldehyde is converted to 1,3-propanediol by an enzyme with hydroxy aldehyde reductase activity.

The inventors of the present invention have now found that by combining this specific biosynthetic pathway for 1,3-propanediol with the overexpression of a gene encoding a pyruvate carboxylase, a significantly improved 1,3-propanediol yield can be obtained. This result was unexpected in view of the prior art, which only describes the expression of pyruvate carboxylase in relation to increasing amino acid production or the production of TCA cycle metabolites.

Accordingly, in a first aspect, the present invention provides a microorganism genetically modified for the bioproduction of 1,3-propanediol, wherein the microorganism comprises:
- a three-step metabolic pathway for the production of 1,3-propanediol, comprising a first step of conversion of L-homoserine to 4-hydroxy-2-ketobutyrate with an enzyme having transaminase activity or oxidase activity or any equivalent enzyme having the capability to convert L-homoserine into 4-hydroxy-2-ketobutyrate, a second step of decarboxylation of 4-hydroxy-2-ketobutyrate to 3-hydroxypropionaldehyde with an enzyme having a hydroxy-2-keto acid decarboxylase activity, and a third step of reduction of 3-hydroxypropionaldehyde to 1,3-propanediol with an enzyme having hydroxy aldehyde reductase activity, and
- overexepression of a gene encoding a pyruvate carboxylase.

Microorganisms according to the present invention, have a significantly increased 1,3-propanediol yield in comparison to microorganisms comprising the three-step metabolic pathway for the production of 1,3-propanediol alone or other microorganisms genetically modified for the production of 1,3-propanediol. In one embodiment, the 1,3-propanediol yield is increased by at least 10 to 20% in comparison to other microorganisms genetically modified for the production of 1,3-propanediol. More preferentially the 1,3-propanediol yield is increased by at least by 30%.

It will be understood that any microorganism known in the art may be useful in the present invention. For example, the microorganism may be selected from the group comprising a bacterium, a yeast or a fungus. In one embodiment, the microorganism is a bacterium selected from the group comprising *Enterobacteriaceae*, *Clostridiaceae*, *Bacillaceae*, *Streptomycetaceae* and *Corynebacteriaceae.*

In one embodiment, the microorganism comprises at least one gene encoding an enzyme having the capability to convert L-homoserine to 4-hydroxy-2-ketobutyrate, one gene encoding an enzyme with hydroxy-2-keto acid decarboxylase activity and one gene encoding an enzyme with hydroxy aldehyde reductase activity. These genes may be exogenous (heterologous or homologous) or endogenous to the microorganism. Furthermore, these genes may be expressed chromosomally or extrachromosomally. In one embodiment, the enzyme having the capability to convert L-homoserine to 4-hydroxy-2-ketobutyrate is encoded by the gene *ser*C from *Escherichia coli.* In one embodiment, the enzyme having hydroxy-2-keto acid decarboxylase activity is encoded by the gene *kivD* from *Lactococcus lactis.* In one embodiment, the enzyme having hydroxy aldehyde reductase activity is encoded by the gene *yqhD* from *Escherichia coli.*

The microorganism according to the present invention is also genetically modified to overexpress a gene encoding a pyruvate carboxylase. In one embodiment, the gene encoding a pyruvate carboxylase is heterologous. In a preferred embodiment of the invention the gene encoding a pyruvate carboxylase is the gene *pyc* from *Rhizobium etli.* This gene may be expressed chromosomally or extrachromosomally. In one embodiment, the heterologous gene encoding a pyruvate carboxylase is carried by a plasmid. In another embodiment, the heterologous gene encoding a pyruvate carboxylase is inserted in the chromosome of the microorganism.

In a particular embodiment, the microorganism further comprises heterologous genes enabling said microorganism to utilize sucrose as a sole carbon source.

The present invention also relates to a method for the fermentative production of 1,3-propanediol, comprising the steps of :
- culturing a microorganism as described above on an appropriate culture medium comprising a source of carbon, and
- recovering 1,3-propanediol from the culture medium.

Accordingly, in a second aspect, the present invention provides a method for the fermentative production of 1,3-propanediol, comprising the steps of :
(a) culturing a microorganism on an appropriate culture medium comprising a source of carbon, wherein said microorganism comprises:
   - a three-step metabolic pathway for the production of 1,3-propanediol, comprising a first step of conversion of L-homoserine to 4-hydroxy-2-ketobutyrate, a second step of decarboxylation of 4-hydroxy-2-ketobutyrate to 3-hydroxypropionaldehyde with an enzyme having a hydroxy-2-keto acid decarboxylase activity, and a third step of reduction of the obtained 3-hydroxypropionaldehyde to 1,3-propanediol with an enzyme having hydroxy aldehyde reductase activity, and
   - overexepression of a gene encoding a pyruvate carboxylase, and
(b) recovering 1,3-propanediol from the culture medium.

### DETAILED DESCRIPTION OF THE INVENTION

Before describing the present invention in detail, it is to be understood that this invention is not limited to particularly exemplified methods and may, of course, vary. It is also to be understood that the terminology used herein is for the purpose of describing particular embodiments of the invention only, and is not intended to be limiting, which will be limited only by the appended claims.

All publications, patents and patent applications cited herein, whether *supra* or *infra*, are hereby incorporated by reference in their entirety. However, publications mentioned herein are cited for the purpose of describing and disclosing the protocols, reagents and vectors that are reported in the publications and that might be used in connection with the invention. Nothing herein is to be construed as an admission that the invention is not entitled to antedate such disclosure by virtue of prior invention.

Furthermore, the practice of the present invention employs, unless otherwise indicated, conventional microbiological and molecular biological techniques within the skill of the art. Such techniques are well known to the skilled worker, and are explained fully in the litterature. See, for example, Prescott *et al.* (1999) and Sambrook *et al.* (1989) (2001).

It must be noted that as used herein and in the appended claims, the singular forms "a", "an", and "the" include plural reference unless the context clearly dictates otherwise. Thus, for example, a reference to "a microorganism" includes a plurality of such microorganisms, and a reference to "an endogenous gene" is a reference to one or more endogenous genes, and so forth. Unless defined otherwise, all technical and scientific terms used herein have the same meanings as commonly understood by one of ordinary skill in the art to which this invention belongs. Although any materials and methods similar or equivalent to those described herein can be used to practice or test the present invention, the preferred materials and methods are now described.

As used herein, the following terms may be used for interpretation of the claims and specification.

In the claims which follow and in the preceding description of the invention, except where the context requires otherwise due to express language or necessary implication, the word "comprise" or variations such as "comprises" or "comprising" is used in an inclusive sense, i.e. to specify the presence of the stated features but not to preclude the presence or addition of further features in various embodiments of the invention.

The term "exogenous gene" means that the gene was introduced into a microorganism, by means well known by the man skilled in the art. Exogenous genes can be heterologous or not.

The term "endogenous gene" means that the gene was present in the microorganism before any genetic modification, in the wild-type strain. Endogenous gene may be overexpressed by introducing heterologous sequences in addition to, or to replace endogenous regulatory elements, or by introducing one or more supplementary copies of the gene into the chromosome or a plasmid.

The term "heterologous gene" means that the gene is derived from a species of microorganism different from the recipient microorganism that expresses it. It refers to a gene which is not naturally occurring in the microorganism.

All genes are referenced with their common names and with references that give access to their nucleotidic sequences on the website http://www.ncbi.nlm.nih.gov/gene.

The present invention relates to a microorganism genetically modified for the bioproduction of 1,3-propanediol. The term "microorganism", as used herein, refers to a bacterium, yeast or a fungus. Preferentially, the microorganism is selected from the group comprising *Enterobacteriaceae*, *Clostridiaceae*, *Bacillaceae*, *Streptomycetaceae* and *Corynebacteriaceae.* More preferentially the microorganism is a species of *Escherichia*, *Clostridium*, *Bacillus*, *Klebsiella*, *Pantoea*, *Salmonella* or *Corynebacterium.* Even more preferentially the microorganism is either the species *Escherichia coli* or *Corynebacterium glutamicum* or *Clostridium acetobutylicum* or *Bacillus subtilis.*

Before describing the present invention in detail, it is to be understood that this invention is not limited to particularly exemplified methods and may, of course, vary. In particular, examples show modified *Escherichia coli* (*E. coli*) strains, but these modifications can easily be performed on other microorganisms of the same family.

*E. coli* belongs to the *Enterobacteriaceae* family, which comprises members that are Gram-negative, rod-shaped, non-spore forming and are typically 1-5 µm in length. Most members have flagella used to move about, but a few genera are non-motile. Many members of this family are a normal part of the gut flora found in the intestines of humans and other animals, while others are found in water or soil, or are parasites on a variety of different animals and plants. *E. coli* is one of the most important model organisms, but other important members of the *Enterobacteriaceae* family include *Klebsiella*, in particular *Klebsiella pneumoniae*; and *Salmonella.*

The term "genetically modified microorganism", as used herein, refers to a microorganism that is not found in nature and is genetically different from equivalent microorganisms found in nature. The microorganism may be modified through either the introduction or deletion of new genetic elements. Further, a microorganism may be modified by forcing the development and evolution of new metabolic pathways by combining directed mutagenesis and evolution under specific selection pressure (see, for example, W02004076659).

A microorganism may be modified to express exogenous genes if these genes are introduced into the microorganism with all the elements allowing their expression in the host microorganism. The modification or "transformation" of microorganisms with exogenous DNA is a routine task for those skilled in the art.

Exogenous genes may be integrated into the host genome, or expressed extrachromosomally by plasmids or vectors. A variety of plasmids, which differ with respect to their origin of replication and their copy number in the cell, are all known in the art. These genes may be heterologous or homologous.

Endogenous genes may also be modified to modulate their expression and/or activity. For example, mutations may be introduced into the coding sequence to modify the gene product or heterologous sequences may be introducted in addition to or to replace endogenous regulatory elements. Modulation of an endogenous gene may result in the upregulation and/or enhancement of the activity of the gene product, or alternatively, down regulate and/or lower the activity of the endogenous gene product. Another way to enhance expression of endogenous genes is to introduce one or more supplementary copies of the gene onto the chromosome or a plasmid.

Important elements for controlling the expression of genes are promoters. In a preferred embodiment of the invention, genes may be expressed using promoters with different strengths, which, in addition, may be inducible. These promoters may be homologous or heterologous. It is well within the ability of the person skilled in the art to select appropriate promoters, for example, the promoters P*trc*, P*tac*, P*lac* or the lambda promoter *cI* are widely used.

As used herein, the following gene or protein names are given for the purpose of describing particular embodiments of the invention only, and are not intended to be limiting if the invention encompasses similar genes and proteins exhibiting the same role or function with another name.

The microorganism of the present invention is modified to express a three-step metabolic pathway for the production of 1,3-propanediol, comprising a first step of conversion of L-homoserine to 4-hydroxy-2-ketobutyrate. This conversion step may be achieved with any enzyme having the capability to convert L-homoserine into 4-hydroxy-2-ketobutyrate. The term 'an enzyme having the capability to convert L-homoserine into 4-hydroxy-2-ketobutyrate', as used herein, refers to an enzyme having L-homoserine as a substrate and 4-hydroxy-2-ketobutyrate as a product. Enzymes having this activity are known in the art and include, for example, homoserine transaminase, homoserine oxidase or homoserine:NAD+ oxidoreductase. For example, increasing the expression of homoserine oxidase can be accomplished by introducing and overexpressing the gene coding for L-amino acid oxidase from *Rhodococcus opacus* or from a *Crotalus sp*., or by introducing mutations into the gene that increase the activity of the corresponding protein. Increasing the level of expression of homoserine transaminase can be accomplished by introducing artificial promoters that drive the expression of the *serC* gene of *E. coli,* by increasing the number of copies of the gene in the cell or by introducing mutations into the *serC* gene that increase the activity of the corresponding protein. In one particular embodiment, the enzyme having the capability to convert L-homoserine into 4-hydroxy-2-ketobutyrate is encoded by a homoserine transaminase. In another embodiment, the homoserine transaminase is encoded by the gene *serC* from *E. coli,* encoding 3-phosphoserine/phosphohydroxythreonine aminotransferase (Reference EcoGene: EG10946).

The second step in the three-step metabolic pathway for the production of 1,3-propanediol comprises the decarboxylation of 4-hydroxy-2-ketobutyrate with an enzyme having a hydroxy-2-keto acid decarboxylase activity. The term 'an enzyme having a hydroxy-2-keto acid decarboxylase activity', as used herein, refers to an enzyme having a decarboxylase activity, whose substrate is a hydroxy-2-keto acid. Genes coding for enzymes having hydroxy-2-keto acid decarboxylase activity are well known in the art and include, for example, *pdc* genes from various species, and more particularly the *pdc1, pdc5, pdc6, aro10* and *thi3* genes from *Saccharomyces cerevisiae*, *kivD* and *kdcA* gene from *Lactococcus lactis*; *pdc* gene from *Clostridium acetobutylicum*; *pdc2* and *pdc3* genes from *Arabidopsis thaliana*; *pdc1*, *pdc2* and *aro10* genes from *Pichia stipitis*; and *pdc* gene from *Zymomonas mobilis.* The first subunit of the 2-ketoglutarate decarboxylase complex, encoded by the gene *sucA* from *Escherichia coli*, also possesses hydroxy-2-keto acid decarboxylase activity, as well as the enzyme encoded by the gene *dxs* of *Escherichia coli.* Functional homologues, functional variants and functional fragments of said genes and proteins are encompassed by the definition.

According to a specific embodiment of the invention, the microorganism comprises an enzyme having hydroxy-2-keto acid decarboxylase activity encoded by an endogenous gene. The microorganism comprising a hydroxy-2-keto acid decarboxylase is preferably selected from the group comprising *Saccharomyces cerevisiae* (Pdc1, Pdc5, Pdc6, Aro10, Thi3); *Lactococcus lactis* (Kivd, KdcA); *Clostridium acetobutylicum* (Pdc); *Arabidopsis thaliana* (Pdc2, Pdc3); *Pichia stipitis* (Pdc1, Pdc2, Aro10); *Zymomonas mobilis* (Pdc); *Mycobacterium tuberculosis.* In one embodiment, the microorganism of the present invention is modified to enhance the expression of the endogenous gene coding for the hydroxy-2-keto acid decarboxylase.

According to another embodiment of the invention, the microorganism does not comprise an efficient endogenous gene coding for an enzyme with hydroxy-2-keto acid decarboxylase activity. Accordingly, in one embodiment, the microorganism of the present invention comprises an enzyme having hydroxy-2-keto acid decarboxylase activity encoded by a heterologous gene. Heterologous genes coding for an enzyme with hydroxy-2-keto acid decarboxylase activity are known by the skilled person and discussed *supra.* According to this specific embodiment of the invention, the microorganism is preferably selected from the group comprising *Escherichia coli*, *Corynebacterium glutamicum* or *Bacillus subtilis.* In one particular embodiment, the enzyme having hydroxy-2-keto acid decarboxylase activity is encoded by the gene *kiv*D from *Lactococcus lactis* (Reference NC_013656).

The third step in the three-step metabolic pathway for the production of 1,3-propanediol comprises the reduction of the obtained 3-hydroxypropionaldehyde with an enzyme having hydroxy aldehyde reductase activity. The term 'an enzyme having a hydroxy aldehyde reductase activity', as used herein, refers to an enzyme having a reductase activity, whose substrate is a hydroxy aldehyde. Genes coding for enzymes having hydroxy aldehyde reductase activity are well known in the art and include, for example, the *yqhD*, *fucO*, *dkgA*, *dkgB* genes from *Escherichia coli* and the ADH1 and ADH2 genes from *Saccharomyces cerevisiae.* Functional homologues, functional variants and functional fragments of said genes and proteins are encompassed by the definition.

According to one embodiment of the invention, the microorganism comprises an enzyme having hydroxy aldehyde reductase activity encoded by an endogenous enzyme. The microorganism comprising an enzyme with hydroxy aldehyde reductase activity may be any microorganism having at least one enzyme having aldehyde reductase activity or alcohol dehydrogenase activity. The microorganism comprising an enzyme with hydroxy aldehyde reductase activity is preferably selected from the group comprising *Escherichia coli* (YqhD, FucO, DkgA, DkgB) and *Saccharomyces cerevisiae* (ADH1, ADH2); and all organisms having at least one enzyme having aldehyde reductase activity or alcohol dehydrogenase activity. This microorganism having endogenous hydroxy aldehyde reductase activity can be further modified to enhance expression of the endogenous gene coding for the hydroxy aldehyde reductase.

According to another embodiment of the invention, the microorganism does not comprise an endogenous gene coding for an enzyme with hydroxy aldehyde reductase activity. The microorganism lacking an endogenous hydroxy aldehyde reductase activity is preferably selected from the group comprising *Corynebacterium glutamicum* or *Bacillus subtilis.* Accordingly, in one embodiment, the microorganism of the present invention comprises an enzyme having hydroxy aldehyde reductase activity encoded by a heterologous gene. Heterologous genes coding for an enzyme with hydroxy aldehyde reductase activity are known by the skilled person and discussed *supra.* In one particular embodiment, the enzyme with hydroxy aldehyde reductase activity is encoded by the gene *yqh*D from *E*. *coli* (Reference EcoGene: EG13014).

The microorganism of the present invention is also genetically modified to overexpress a gene encoding a pyruvate carboxylase, which creates a stimulated flux in the oxaloacetate biosynthesis pathway and an oxaloacetate pool. The term "overexpression of a gene encoding a pyruvate carboxylase" refers either to overexpression of an endogenous gene or to expression of an exogenous gene. As disclosed *supra,* an endogenous gene may be overexpressed by introducing heterologous sequences in addition to or to replace endogenous regulatory elements or by introducing one or more supplementary copies of the gene onto the chromosome or a plasmid. Exogenous genes may be integrated into the host genome, or expressed extrachromosomally by plasmids or vectors. A variety of plasmids, which differ with respect to their origin of replication and their copy number in the cell, are all known in the art.

Important elements for controlling the expression of genes are promoters. In a preferred embodiment of the invention, genes may be expressed using promoters with different strengths, which, in addition, may be inducible. These promoters may be homologous or heterologous. It is well within the ability of the person skilled in the art to select appropriate promoters, for example, the promoters P*trc,* P*tac,* P*lac* or the lambda promoter *cI* are widely used.

In a specific embodiment of the invention, the gene encoding a pyruvate carboxylase is heterologous. The heterologous gene encoding pyruvate carboxylase may be any appropriate bacterial pyruvate carboxylase gene, preferentially the *pyc* gene of *Rhizobium etli*, *Pseudomonas fluorescens*, *Corynebacterium glutamicum* or *Bacillus subtilis.* In one embodiment, the heterologous gene encoding a pyruvate carboxylase is the *pyc* gene of *Rhizobium etli*, reference NC_007761. The heterologous pyruvate carboxylase gene may be expressed chromosomally, ie. inserted into the chromosome of the microorganism, or extrachromosomally, ie. carried by a plasmid. Methods for expressing heterologous genes in microorganisms are well known to persons skilled in the art and are also discussed *supra* and *infra.*

Microorganisms having the above discussed genetic modifications have an increased 1,3-propanediol yield in comparison to microorganisms comprising the three-step metabolic pathway for the production of 1,3-propanediol alone or other microorganisms genetically modified for the production of 1,3-propanediol. Specifically, 1,3-propanediol yield in microorganisms of the present invention is increased by at least 10% in comparison to other microorganisms genetically modified for the production of 1,3-propanediol not overexpressing pyruvate carboxylase.

In addition to the modifications described above, the microorganism may also contain a number of other optional modifications.

In one embodiment, the microorganism has a stimulated flux into the homoserine biosynthesis pathway. This can be achieved, for example, by increasing the expression of aspartokinase and homoserine dehydrogenase and/or aspartate semialdehyde dehydrogenase, encoded by the *thrA*/*metL* and *asd* genes, respectively. Increasing the expression of aspartokinase and homoserine dehydrogenase and/or aspartate semialdehyde dehydrogenase can be accomplished by introducing strong artificial or natural promoters that drive the expression of the *thrA*/*metL* and/or *asd* genes, by increasing the number of copies in the cell or by introducing mutations into the *thrA* and/or *asd* genes that increase the activity of the corresponding proteins.

In a particular embodiment of the invention, mutations can be introduced into the *thrA* gene to reduce its sensitivity to the feed-back inhibitor threonine (feed-back desensitized alleles) and thus permit an increased activity in the presence of threonine.

In a further embodiment of the invention, the microorganism is modified such that homoserine conversion to compounds other than 1,3-propanediol is attenuated. This result may be achieved by attenuating the level of homoserine consuming enzymes, for example, homoserine kinase and threonine synthase (encoded by *thrB* and *thrC*) and homoserine O-transsuccinylase (encoded by *metA*). For example, these genes can be attenuated by replacing the natural promoter by a weaker promoter or by inserting elements that destabilize the corresponding messenger RNA or protein. Further, if desired, complete attenuation of the gene can be achieved by the deletion of the corresponding DNA sequence.

In a further embodiment of the invention, the microorganism is modified such that homoserine precursor conversion to compounds other than 3-hydroxypropionate is attenuated. This result may be achieved by attenuating the level of dihydrodipicolinate synthase (encoded by *dapA*). Attenuation of *dapA* may be achieved, for example, by replacing the natural promoter by a lower strength promoter or by inserting elements that destabilize the corresponding messenger RNA or the protein. If desired, complete attenuation of the gene can also be achieved by a deletion of the corresponding DNA sequence.

In a further embodiment of the invention, the microorganism is modified such that 3-hydroxypropionaldehyde conversion to compounds other than 1,3-propanediol is attenuated. This may be achieved by attenuating the level of 3-hydroxypropionaldehyde consuming enzymes, for example, 3-hydroxypropionaldehyde dehydrogenase (encoded by *aldA*, *aldB*, *aldH*). These genes can be attenuated by replacing the natural promoter with a weaker promoter or by inserting elements that destabilize the corresponding messenger RNA or the protein. If desired, complete attenuation of the gene can also be achieved by the deletion of the corresponding DNA sequence.

In a further embodiment of the invention, the microorganism further comprises exogenous genes enabling said microorganism to utilize sucrose as a sole carbon source. The term 'able to utilize sucrose as sole carbon source', as used herein, indicates that the microorganism can grow in a medium containing sucrose as unique carbon source; however, it is understood that the culture medium may comprise additional minor carbon sources in addition to sucrose such as hexoses (such as glucose, fructose, galactose or lactose), pentoses, monosaccharides, disaccharides (such as sucrose, cellobiose or maltose), oligosaccharides, starch or its derivatives, hemicelluloses, glycerol and combinations thereof.

In a specific embodiment of the invention, the microorganism comprises functional genes coding for a PTS sucrose utilization system and/or for a non-PTS sucrose utilization system.

A PTS sucrose utilization system is a system for sucrose utilization based on the transport of sucrose by a phosphoenolpyruvate (PEP)-dependent sucrose phosphotransferase system (Sucrose-PTS). A phosphotransferase system couples the transport of a sugar (e.g. sucrose or glucose) with the phosphorylation of the sugar using PEP as phosphate donor. After transport into the cell, the sucrose-phosphate is cleaved into glucose-6-phosphate and fructose by an invertase. Fructose is then phosphorylated into fructose-6-phosphate by a fructokinase. The genes coding for this PTS sucrose utilization system can be controlled by a regulatory protein.

A non-PTS sucrose utilization system is a system for sucrose utilization based on transport of sucrose by a system independent of phosphoenolpyruvate. After transport into the cell, the sucrose is cleaved into glucose and fructose by an invertase. Fructose is then phosphorylated into fructose-6-phosphate by a fructokinase and glucose is phosphorylated into glucose-6-phosphate by a glucokinase. The genes coding for this non-PTS sucrose utilization system can be controlled by a regulatory protein.

In a specific aspect of the invention, the microorganism expresses naturally or has been modified with the introduction of the genes: *scr*KYABR (*scr*K coding for a fructokinase, *scr*Y coding for a porin, *scr*A coding for the Protein IIBC, scrB coding for a sucrose-6-P invertase, *scr*R coding for a repressor) from *Salmonella.* A conjugative plasmid pUR400 bearing said genes *scr*KYABR might be used to transform the microorganism. These genes can be used all together in combination, or in any combination comprising at least one of these genes. In particular, the gene *scr*R can be omitted.

In another specific aspect of the invention, the microorganism expresses naturally or has been modified with the introduction of the genes from *E. coli* EC3132 i.e. the genes *csc*BKAR coding for a sucrose proton symport transport system (*csc*B), a fructokinase (*csc*K), an invertase (*csc*A) and a sucrose-specific repressor (*csc*R). These genes can be used all together in combination or in any combination comprising at least one of these genes. In particular, the gene *csc*R can be omitted. Homologous genes from other organisms can also be used.

The designation of these genes has a more general meaning according to the invention and covers the corresponding genes in other micro-organisms. Using the GenBank references of the genes from *Salmonella or* from *E. coli,* those skilled in the art can determine equivalent genes in organisms other than *Salmonella* or *E. coli.*

The means of identification of the homologous sequences and their percentage homologies are well-known to those skilled in the art, and include in particular the BLAST programmes that can be used on the website http://www.ncbi.nlm.nih.gov/BLAST/ with the default parameters indicated on that website. The sequences obtained can be exploited (aligned) using for example the programmes CLUSTALW (httL)://,www.ebi.ac.uk/cluatalw/), with the default parameters indicated on these websites.

The PFAM database (protein families database of alignments and hidden Markov models http://www.sanger.ac.uk/Software/Pfam/) is a large collection of alignments of protein sequences. Each PFAM makes it possible to visualise multiple alignments, view protein domains, evaluate distributions among organisms, gain access to other databases and visualise known protein structures.

COGs (clusters of orthologous groups of proteins http://www.ncbi.nlm.nih.gov/COG/) are obtained by comparing protein sequences derived from 66 fully sequenced unicellular genomes representing 14 major phylogenetic lines. Each COG is defined from at least three lines, making it possible to identify ancient conserved domains.

All techniques for transforming the microorganisms, and regulatory elements used for enhancing production of 1,3-propanediol are well known in the art and available in the literature, including applicant's own patent applications on modification of biosynthesis pathways in various microorganisms, including WO 2008/052973, WO 2008/052595, WO 2008/040387, WO 2007/144346, WO 2007/141316, WO 2007/077041, WO 2007/017710, WO 2006/082254, WO 2006/082252, WO 2005/111202, WO 2005/073364, WO 2005/047498, WO 2004/076659, the content of which is incorporated herein by reference.

The microorganism, as described above, is useful in a method for the fermentative production of 1,3-propanediol. Fermentation is generally conducted in fermenters with an appropriate culture medium adapted to the microorganism, and if necessary co-substrates. In one embodiment, the fermentative production of 1,3-propanediol, comprising the steps of: culturing a microorganism according to any one of claims 1 to 13 on an appropriate culture medium comprising a source of carbon, and recovering 1,3-propanediol from the culture medium.

The term an 'appropriate culture medium', as used herein, refers to a medium (e.g., a sterile, liquid media) comprising nutrients essential or beneficial to the maintenance and/or growth of the cell such as carbon sources or carbon substrate, nitrogen sources, for example, peptone, yeast extracts, meat extracts, malt extracts, urea, ammonium sulfate, ammonium chloride, ammonium nitrate and ammonium phosphate; phosphorus sources, for example, monopotassium phosphate or dipotassium phosphate; trace elements (e.g., metal salts), for example magnesium salts, cobalt salts and/or manganese salts; as well as growth factors such as amino acids, vitamins, growth promoters, and the like.

As an example of known culture media for *E. coli,* the culture medium can be of identical or similar composition to an M9 medium (Anderson, 1946), an M63 medium (Miller, 1992) or a medium such as defined by Schaefer *et al*. (1999).

The term "source of carbon" according to the invention denotes any source of carbon that can be used by those skilled in the art to support the normal growth of a microorganism, which can be hexoses such as glucose, galactose or lactose; pentoses; monosaccharides; disaccharides such as sucrose, cellobiose or maltose; oligosaccharides; molasses; starch or its derivatives; hemicelluloses; glycerol and combinations thereof. An especially preferred carbon source is glucose. Another preferred carbon source is sucrose.

In a particular embodiment of the invention, the carbon source is derived from renewable feed-stock. Renewable feed-stock is defined as raw material required for certain industrial processes that can be regenerated within a brief delay and in sufficient amount to permit its transformation into the desired product. Vegetal biomass treated or not, is an interesting renewable carbon source.

The culture conditions for the fermentation process can be readily defined by those skilled in the art. In particular, bacteria are fermented at temperatures between 20°C and 55°C, preferably between 25°C and 40°C, and preferably at about 35°C for *Clostridiaceae* and at about 37°C for *Enterobacteriaceae.*

The terms 'cultivating', 'culture', 'growth' and 'fermentation', as used herein, are used interchangeably to denote the growth of bacteria in an appropriate growth medium containing a simple carbon source. Fermentation is a classical process that can be performed under aerobic, microaerobic or anaerobic conditions.

The term 'under aerobic conditions', as used herein, indicates that oxygen is provided to the culture by dissolving the gas into the liquid phase. This could be obtained by (1) sparging oxygen containing gas (e.g. air) into the liquid phase or (2) shaking the vessel containing the culture medium in order to transfer the oxygen contained in the head space into the liquid phase. Fermentation under aerobic conditions instead of anaerobic conditions has the advantage of providing oxygen as an electron acceptor, which improves the capacity of the strain to produce more energy in form of ATP for cellular processes. Accordingly, the strain generally has improved metabolism.

Micro-aerobic conditions are defined as culture conditions wherein low percentages of oxygen (e.g. using a mixture of gas containing between 0.1 and 10% of oxygen, completed to 100% with nitrogen), is dissolved into the liquid phase.

Anaerobic conditions are defined as culture conditions wherein no oxygen is provided to the culture medium. Strictly anaerobic conditions are obtained by sparging an inert gas like nitrogen into the culture medium to remove traces of other gas. Nitrate can be used as an electron acceptor to improve ATP production by the strain and improve its metabolism.

Recovering 1,3-propanediol produced by the microorganism and methods of the present invention from culture medium is a routine task for a person skilled in the art. In one embodiment, the recovered 1,3-propanediol is further purified. Methods for recovery and purification are disclosed in the following patent applications: WO 2009/068110 and WO 2010/037843 which are incorporated herein by reference.

### DRAWINGS

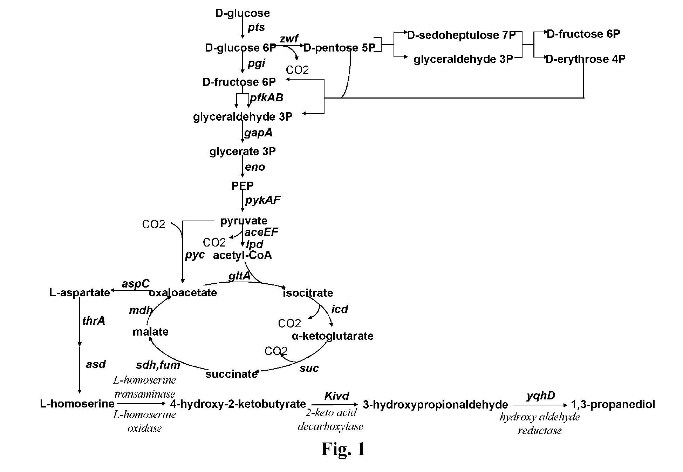

### Figure 1. Biosynthesis pathway for 1,3-propanediol

### EXAMPLES

### Exemple 1: Protocoles

Several protocols have been used to construct 1,3-propanediol producing strains and are described in the following examples.

### Protocol 1: Chromosomal modifications by homologous recombination and selection of recombinants (Datsenko & Wanner, 2000)

Allelic replacement or gene disruption in specified chromosomal loci was carried out by homologous recombination as described by Datsenko & Wanner (2000). The chloramphenicol (Cm) resistance *cat* and the kanamycin (Km) resistance *kan*, flanked by Flp recognition sites, were amplified by PCR by using pKD3 or pKD4 plasmids as template respectively. The resulting PCR products were used to transform the recipient *E*. *coli* strain harbouring plasmid pKD46 that expresses the λ Red (γ, β, exo) recombinase. Antibiotic-resistant transformants were then selected and the chromosomal structure of the mutated *loci* was verified by PCR analysis with the appropriate primers listed in Table 2. The *cat* and *kan*-resistance genes were removed by using plasmid pCP20 as described by Datsenko & Wanner (2000). Antibiotic sensitive clones were then verified by PCR using primers listed in Table 2

### Protocol 2: Transduction of phage P1

Chromosomal modifications were transferred to a given *E*. *coli* recipient strain by P1 transduction. The protocol is composed of 2 steps: (i) preparation of the phage lysate on a donor strain containing the resistance associated chromosomal modification and (ii) infection of the recipient strain by this phage lysate.

### Preparation of the phase lysate

- Inoculate 100 µl of an overnight culture of the strain MG1655 with the chromosomal modification of interest in 10 ml of LB + Cm 30µg/ml or Km 50µg/ml + glucose 0.2% + CaCl₂ 5 mM.
- Incubate 30 min at 37°C with shaking.
- Add 100 µl of P1 phage lysate prepared on the donor strain MG1655 (approx. 1 x 10⁹ phage/ml).
- Shake at 37°C for 3 hours until the complete lysis of cells.
- Add 200 µl of chloroform, and vortex
- Centrifuge 10 min at 4500 g to eliminate cell debris.
- Transfer of supernatant to a sterile tube.
- Store the lysate at 4°C.

### Transduction

- Centrifuge 10 min at 1500 g 5 ml of an overnight culture of the *E*. *coli* recipient strain cultivated in LB medium.
- Suspend the cell pellet in 2.5 ml of MgSO₄ 10 mM, CaCl₂ 5 mM.
- Infect 100 µl cells with 100 µl P1 phage of strain MG1655 with the modification on the chromosome (test tube) and as a control tubes 100 µl cells without P1 phage and 100 µl P1 phage without cells.
- Incubate 30 min at 30°C without shaking.
- Add 100 µl sodium citrate 1 M in each tube, and vortex.
- Add 1 ml of LB.
- Incubate 1 hour at 37°C with shaking
- Centrifuge 3 min at 7000 rpm.
- Plate on LB + Cm 30 µg/ml or Km 50 µg/ml
- Incubate at 37°C overnight.

The antibiotic-resistant transductants were then selected and the chromosomal structure of the mutated locus was verified by PCR analysis with the appropriate primers listed in Table 2.

**Table 1: The genotype and corresponding number of intermediate strains and producer strains that appear in the following examples.**

| **Strain number** | **Genotype** |
|---|---|
| **1** | MG1655 Δ*pyk*F Δ*met*A Δ*thr*LABC (pBBR1MCS5-Ptrc01/RBS01*2-*ygh*D-*kiv*D11-TT07) (pME101-*thr*A*1-*ser*C) |
| **2** | MG1655 Δ*pyk*F Δ*met*A Δ*thr*LABC Δ*melB::TT02*-*Ptrc01*/*ARN01*/*RBS01***2-pycre-TT07::Km* (pBBRIMCS5-Ptrc01/RBS01*2-*yqh*D-*kiv*D11-TT07) (pME101-*thr*A*1-*ser*C) |

**Table 2: Primers used for PCR verifications of chromosomal modifications described above**

| **Genes name** | **Primers name** | **SEQ ID N°** | **Location of the homology with the chromosomal region** | **Sequences** |
|---|---|---|---|---|
| ***melB*** | Ome 1845- DmelB-verif1-F | 1 | 4340168-4340187 | GCCGATTTTGTCGTGGTGGC |
| | Ome 1846-DmelB-verif2-R DmelB-venf2-R | 2 | 4344044-4344065 | GCCGGTTATCCATCAGGTTC AC |

### Example 2: Construction of strain 2 : MG1655 ΔpykF ΔmetA ΔthrLABC ΔmelB::TT02-Ptrc01/ARN01/RBS01*2-pycre-TT07::Km (pBBR1MCS5-Ptrc01/RBS01*2-yqhD-kivD11-TT07) (pME101-thrA*1-serC)

### 1. Construction of plasmid pUC18-TTadc-CI*0-PlambdaR*(-35)-ΔmelB::TT02-TTadc-Ptrc01/ARN01/RBS01*2-pycre-TT07::Km

Plasmid pUC18-*TTade-CI*0-*P*lambdaR*(-35)-*Δ*melB::TT02-TTadc-Ptrc01*/*ARN01*/*RBS01*2-pycre::Km* is derived from pSCB*-Ptrc01*/*ARN01*/*RBS01*2-pycre-*TT07 and pUC18-*TTadc-CI*0-PlambdaR*(-35)-*Δ*melB::TT02-SMC-Km* described below.

### Construction of plasmid pUC18-TTadc-CI*0-PlambdaR*(-35)-ΔmelB::TT02-SMC-Km

To construct plasmid pUC18-*TTadc-CI*0-PlambdaR*(-35)-*Δ*melB::TT02-SMC-Km,* the FRT-Km-FRT resistance cassette was amplified by PCR with primers Ome 1605-K7_Km_ampl_SmaI_BstZ17I_F (SEQ ID N°3) and Ome 1606-K7_Km_ampl_HindIII_R (SEQ ID N°4) using pKD4 as template. The PCR product was digested and cloned between the *BstZ*17I and *Hind*III sites of the pUC18*-TTadc-Cl*0-PlambckrR*(-35)-*Δ*melB::TT02-SMC* described in PCT/EP2010/069473 patent application. The resulting plasmid was verified by DNA sequencing and called pUC18*-TTadc-CI*0-PlambdaR*(-35)-*Δ*melB::TT02-SMC* -Km.

Ome 1605-K7_Km_ampl_SmaI_BstZ17I_F (SEQ ID N°3)
TCCCCCGGGGTATACCATATGAATATCCTCCTTAG
with
- underlined upper case sequence for *Sma*I and *BstZ*17I restriction sites and extrabases,
- upper case sequence corresponding to site primer 1 of plasmid pKD4 (Datsenko, K.A. & Wanner, B.L., 2000, PNAS, 97: 6640-6645)

Ome 1606-K7_Km_ampl_HindIII_R (SEQ ID N°4)
GCCCAAGCTTTGTAGGCTGGAGCTGCTTCG
with
- underlined upper case sequence for *Hind*III restriction site and extrabases,
- upper case sequence corresponding to site primer 2 of plasmid pKD4 (Datsenko & Wanner, 2000)

### Construction of plasmid pSCB-Ptrc01/ARN01/RBS01*2-pycre-TT07

To construct plasmid pSCB-*Ptrc01*/*ARN01*/*ABS01*2-pycre*-TT07, the *Ptrc01*/*ARN01*/*RBS01*2-pycre-TT07* insert was amplified by PCR with primers Ome 2068-SmaI-AvrII-TT07-NotI-pycre-R (SEQ ID N°5) and Ome 2069-PacI-Ptrc01-ARN01-RBS01*2-pycre-F (SEQ ID N°6) using pJB137-P*gapA-pycRe* described in PCT/FR2010/052938 patent application as template and cloned in the pSCB (Stratagene). The resulting vector was verified by sequencing and named pSCB-*Ptrc01*/*ARN01*/*RBS01*2-pycRe*-TT07.

Ome 2068- SmaI-AvrII-TT07-NotI-pycre-R (SEQ ID N°5) 
CGACCCGGGCCTAGGGCAGAAAGGCCCACCCGAAGGTGAGCCAGTGTGAgc
ggccgcTCATCCGCCGTAAACCGCCAGCAGG
 with
- underlined upper case sequence for *Sma*I and *Avr*II restriction sites and extrabases,
- bold upper case for T7Te transcriptional terminator sequence from T7 phage (Harrington *et al*., 2001),
- underlined lower case sequence for *Not*I restriction site,
- italic upper case sequence homologous to the end (4240352 to 4240328) of the *pyc* gene from *Rhizobium etli* CFN42 (reference sequence on the website http://blast.ncbi.nlm.nih.govBlast.cgi),

Ome 2069-PacI-Ptrc01-ARN01-RBS01*2-pycre-F (SEQ ID N°6) 
CGTAGTTAACTTAATTAAGAGCTGTTGACAATTAATCATCCGGCTCGTATAA
TGTGTGGAAGGTGGAGTTATCTCGAGTGAGATATTGTTGACGTAAGGAGGTTAT
AAATGCCCATATCCAAGATACTCGTTGCCAATCG
 with
- underlined upper case sequence for *Hpa*I and *Pac*I restriction sites and extrabases,
- bold upper case sequence for the *trc* promoter sequence (Amman et al., 1983)
- italic and underlined upper case sequence RNA stabilizing sequence (Carrier et al., 1999),
- bold and underlined upper case sequence corresponding to RBS01 sequence with a *Psi*I restriction site
- italic upper case sequence homologous to the beginning (4236889 to 4236919) of the *pyc* gene from *Rhizobium etli* NC_007761

### Construction of plasmid pUC18-TTadc-CI*0-PlambdaR*(-35)-ΔmelB::TT02-TTadc-Ptrc01/ARN01/RBS01*2-pycre-TT07::Km

To construct pUC18-*TTadc-CI*0-*P*lambdaR*(-35)-*Δ*melB::TT02-TTadc-Ptrc01*/*ARN01*/*RBS01*2-pycre-TT07::Km*, the *PacI* / *AvrII* fragment *Ptrc01*/*ARN01*/*RBS01*2-pycre* isolated from pSCB-*Ptrc01*/*ARN01*/*RBS01*2-pycre*-TT07 described above was cloned between the *PacI* / *AvrII* sites of plasmid pUC18*-TTadc-CI*0-PlambdaR*(-35)-*Δ*melB::TT02-SMC-*Km described above. The resulting plasmid was verified by DNA sequencing and called pUC18-*TTadc-CI*0-*P*lambdaR*(-35)-*Δ*melB::TT02-TTadc-Ptrc01*/*ARN01*/*RBS01*2-pycre-TT07::Km.*

### 2. Construction of strain MG1655 ΔmelB::TT02-Ptrc01/ARN01/RBS01*2-pycre-TT07::Km pKD46

To replace the *melB* gene by the *TT02-Ptrc01*/*ARN01*/*RBS01*2-pycre-TT07::Km* region, pUC18-*TTadc-CI*0-*P*lambdaR*(-35)-*Δ*melB::TT02-TTadc-Ptrc01*/*ARN01*/*RBS01*2-pycre-TT07::Km* was digested by *Kpn*I and *Sca*I restriction enzymes and the remaining digested fragment Δ*melB::TT02-TTadc-Ptrc01*/*ARN01*/*BS01*2-pycre-TT07::Km* was introduced into strain MG1655 pKD46 according to Protocol 1.

Kanamycin resistant recombinants were selected and the presence of the Δ*melB::TT02-TTadc-Ptrc01*/*ARN01*/*RBS01*2-pycre-TT07::Km* chromosomal modification was verified by PCR with primers Ome 1845-DmelB-verif1-F (SEQ ID N°1) and Ome 1846-DmelB-verif2-R (SEQ ID N°2) (Table 2) and by DNA sequencing. The verified and selected strain was called MG1655 Δ*melB::TT02-TTadc-Ptrc01*/*ARN01*/*RBS01*2-pycre-TT07::Km* (pKD46).

### 3. Transduction of ΔmelB::TT02-TTadc-Ptrc01/ARN01/RBS01*2-pycRe::Km into strain 1

The Δ*melB::TT02-TTadc-Ptrc01*/*ARN01*/*RBS01*2pycre-TT07::Km* chromosomal modification was transduced into strain 1 described in US61/361.455 patent application, with a P1 phage lysate from strain MG1655 Δ*melB::TT02-TTadc-Ptrc01*/*ARN01*/*RBS01*2-pycre-TT07::Km* (pKD46) described above, according to Protocol 2.

Kanamycin resistant transductants were selected and the presence of the Δ*melB::TT02-TTadc-Ptrc01*/*ARN01*/*RBS01*2-pycre-TT07::Km* chromosomal modification was verified by PCR with Ome 1845-DmelB-verifl-F (SEQ ID N°1) and Ome 1846-DmelB-verif2-R (SEQ ID N°2) (Table 2). The resulting strain MG1655 Δ*pyk*F Δ*met*A Δ*thr*LABC Δ*melB::TT02-TTadc*-Ptrc01/*ARN01*/*RBS01*2-pycre-TT07::Km* (pBBR1MCS5-Ptrc01/RBS01*2-*yqh*D-*kiv*D11-TT07) (pME101-*thr*A*1-*ser*C) was called strain 2.

### Example 3: Shake flask cultures of strains 1 & 2

Production strains were evaluated in small Erlenmeyer flasks using modified M9 medium (Anderson, 1946, Proc. Natl. Acad. Sci. USA 32:120-128) that was supplemented with 4.5 mM threonine, 5 mM methionine, 10 g.L⁻¹ MOPS and 10 g.L⁻¹ glucose and adjusted to pH 6.8.

A 5 mL preculture was grown at 37°C for 6.5 hours in a mixed medium (10 % LB medium (Sigma 25 %) with 2.5 g.L⁻¹ glucose and 90 % minimal medium described above). It was used to inoculate a 50 mL culture to an OD₆₀₀ of 0.1 in minimal medium. IPTG (100 µM) was also added for the induction of the genes on the expression vector pME101. When necessary, antibiotics were added at concentrations of 50 mg.L⁻¹ for kanamycin and spectinomycin and 10 mg.L⁻¹ for gentamycin. The temperature of the cultures was 37°C. When the culture had reached an OD₆₀₀ of 7 to 9, extracellular metabolites were analyzed using HPLC with refractometric detection (organic acids and glucose). Production of 1,3-propanediol was determined by LC/MS/MS. For each strain, two repetitions were made.

**Table 3: PDO production in batch culture by the different strains. SD denotes the standard deviation for the concentration which was calculated on the basis of two repetitions.**

| **Strain** | **PDO (mM)** | **SD** |
|---|---|---|
| Strain 1 | 0.13 | 0.01 |
| Strain 2 | 0.16 | 0.03 |

As can be seen in the table above, PDO production is increased upon overexpression of the *pyc* gene.

### Example 4: Enzymatic activities for pyruvate carboxylase

### Crude extract preparation:

About 25 mg of *E*. *coli* biomass were resuspended in 1 ml of 100 mM Potassium phosphate pH 7, 0.1 mM DTT, 0.1mM pyridoxal 5'-phosphate (PLP) and a protease inhibitor cocktail (Roche). The cells were disrupted using 0.1mm glass beads in a Precelllys 24 homogenizator (Bertin Technologies) for 1 × 30 seconds at 6500 rpm. After sonication, cells debris were removed by centrifugation at 12000g for 30 min at 4°C. The crude extracts were desalted using 2ml Zeba Spin Desalting Columns (Pierce).

### Pyruvate carboxylase activity assay

The pyruvate carboxylase activity was measured at 30°C using a coupled enzymatic assay. The pyruvate carboxylase activity assay was carried out with 100 mM Tris buffer pH 8, 5µM ATP, 10mM MgCl₂, 10 mM KHCO₃, 0.5mM Acetyl-CoA, 0.2 mM NADH, 24 units/ml malate dehydrogenase, 2 mM Pyruvate and about 10 µg of crude extract in a total volume of 1 ml. The consumption of NADH was monitored at 340 nm on a spectrophotometer. The activity detected in control assay, lacking the substrate, was subtracted from the activity detected in the assay with substrate. A unit of pyruvate carboxylase activity is the amount of enzyme required to catalyze the carboxylation of 1 µmol of pyruvate per min at 30°C. (Epsilon 340 nm = 6220 M-1 cm-1)

**Table 4: Activity of crude extracts**

| | **Activity of crude extracts (mUI/mg)** |
|---|---|
| Strain 1 | **<LQ** |
| Strain 2 | **274 ± 15** |

The pyruvate carboxylase activity measured in the crude extract of the strain 1 is inferior to the limit of quantification which is equal to 5 mUI/mg.

### REFERENCES

- Amann E., Brosius J., Ptashne M. (1983), Gene. 25: 167-178
- Anderson EH (1946), Proc. Natl. Acad Sci. USA 32:120-128
- Carrier T.A., Keasling J.D. (1999), Biotechnol. Prog. 15:58-64
- Datsenko KA, Wanner BL (2000), Proc Natl Acad Sci U S A. 97: 6640-6645
- Harrington KJ, Laughlin RB, Liang S (2001), Proc Natl Acad Sci U S A. 98: 5019-5024
- Miller (1992), A Short Course in Bacterial Genetics: A Laboratory Manual and Handbook for Escherichia coli and Related Bacteria, Cold Spring Harbor Laboratory Press, Cold Spring Harbor, New York
- Prescott et al. (1999), "Microbiology" 4th Edition, WCB McGraw-Hill
- Sambrook et al. (1989) (2001), "Molecular Cloning: A Laboratory Manual" 2nd & 3rd Editions, Cold Spring Harbor Laboratory Press
- Schaefer U, Boos W, Takors R, Weuster-Botz D., (1999), Anal. Biochem. 270: 88-96

## Claims

1. A microorganism genetically modified for the bioproduction of 1,3-propanediol, wherein said microorganism comprises:
- a three-step metabolic pathway for the production of 1,3-propanediol, comprising a first step of conversion of L-homoserine to 4-hydroxy-2-ketobutyrate, a second step of decarboxylation of 4-hydroxy-2-ketobutyrate to 3-hydroxypropionaldehyde with an enzyme having a hydroxy-2-keto acid decarboxylase activity, and a third step of reduction of 3-hydroxypropionaldehyde to 1,3-propanediol with an enzyme having hydroxy aldehyde reductase activity, and
- an overexpressed gene encoding a pyruvate carboxylase.

2. The microorganism according to claim 1, wherein the gene encoding pyruvate carboxylase is an heterologous *pyc* gene from *Rhizobium etli*, *Pseudomonas flurescens*, *Corynebacterium glutamicum* or *Bacillus subtilis.*

3. The microorganism according to claim 2, wherein the heterologous gene encoding a pyruvate carboxylase is carried by a plasmid.

4. The microorganism according to claim 2, wherein the heterologous gene encoding a pyruvate carboxylase is inserted in the chromosome of the microorganism.

5. The microorganism according to any one of claims 1 to 4, wherein the enzyme having hydroxy-2-keto acid decarboxylase activity is encoded by an endogenous gene.

6. The microorganism according to any one of claims 1 to 4, wherein the enzyme having hydroxy-2-keto acid decarboxylase activity is encoded by a heterologous gene.

7. The microorganism according to any one of claims 1 to 6, wherein the enzyme having hydroxy-2-keto acid decarboxylase activity is encoded by the gene *kivD* from *Lactococcus lactis.*

8. The microorganism according to any one of claims 1 to 7, wherein the enzyme having hydroxy aldehyde reductase activity is encoded by an endogenous gene.

9. The microorganism according to any one of claims 1 to 7, wherein the enzyme having hydroxy aldehyde reductase activity is encoded by a heterologous gene.

10. The microorganism according to any one of claims 1 to 9, wherein the enzyme having hydroxy aldehyde reductase activity is encoded by the gene *yqhD* from *Escherichia coli.*

11. The microorganism according to any one of claims 1 to 10, wherein said microorganism further comprises heterologous genes enabling said microorganism to utilize sucrose as a sole carbon source.

12. The microorganism according to any one of claims 1 to 11, wherein said microorganism is selected from the group comprising bacterium, yeast and fungus.

13. The microorganism of any one of claims 1 to 12, wherein said microorganism is selected from the group comprising *Enterobacteriaceae*, *Clostridiaceae*, *Bacillaceae*, *Streptomycetaceae* and *Corynebacteriaceae.*

14. A method for the fermentative production of 1,3-propanediol, comprising the steps of :
- culturing a microorganism according to any one of claims 1 to 13 on an appropriate culture medium comprising a source of carbon, and
- recovering 1,3-propanediol from the culture medium.

15. The method of claim 14, wherein said recovered 1,3-propanediol is further purified.


SEQUENCE LISTING

<110>  METABOLIC EXPLORER
       BOISART, Cedric

<120>  Method for the preparation of 1,3-propanediol

<130>  D29434

<160>  6

<170>  PatentIn version 3.5

<210>  1
<211>  20
<212>  DNA
<213>  Artificial Sequence

<220>
<223>  Oligonucleotide

<400>  1
gccgattttg tcgtggtggc                                                   20


<210>  2
<211>  22
<212>  DNA
<213>  Artificial Sequence

<220>
<223>  Oligonucleotide

<400>  2
gccggttatc catcaggttc ac                                                22


<210>  3
<211>  35
<212>  DNA
<213>  Artificial Sequence

<220>
<223>  Oligonucleotide

<400>  3
tcccccgggg tataccatat gaatatcctc cttag                                  35


<210>  4
<211>  30
<212>  DNA
<213>  Artificial Sequence

<220>
<223>  Oligonucleotide

<400>  4
gcccaagctt tgtaggctgg agctgcttcg                                        30


<210>  5
<211>  82
<212>  DNA

<213>  Artificial Sequence

<220>
<223>  Oligonucleotide

<400>  5
cgacccgggc ctagggcaga aaggcccacc cgaaggtgag ccagtgtgag cggccgctca      60

tccgccgtaa accgccagca gg                                                82

<210>  6
<211>  140
<212>  DNA
<213>  Artificial Sequence

<220>
<223>  Oligonucleotide

<400>  6
cgtagttaac ttaattaaga gctgttgaca attaatcatc cggctcgtat aatgtgtgga      60

aggtggagtt atctcgagtg agatattgtt gacgtaagga ggttataaat gcccatatcc     120

aagatactcg ttgccaatcg                                                  140